# EUROPEAN PATENT SPECIFICATION

(11) **EP 4 162 867 B1**
(45) Date of publication and mention of the grant of the patent: **22.01.2025**
(21) Application number: 22211084.3
(22) Date of filing: 03.06.2019
(51) Int. Cl.: A61B 5/00, G10K 11/178, A61F 11/14, A61F 11/08, A61F 11/06, A61B 5/11, A61B 5/12

(54) **HEARING PROTECTION SYSTEM FOR A DOG**
HUNDGEHÖRSCHUTZSYSTEM
SYSTÈME DE PROTECTION AUDITIVE POUR UN CHIEN

(30) Priority: 04.06.2018 US 201862679982 P; 02.06.2019 US 201962856070 P
(43) Date of publication of application: 12.04.2023
(62) Divisional of application: 19847869.5
(73) Proprietor: Zeteo Tech, Inc., Sykesville, MD 21784 (US)
(72) Inventor: McLoughlin, Michael, Sykesville, MD 21784 (US)
(74) Representative: Körfer, Thomas

(56) References cited:
- WO-A1-2016/129731
- US-A1- 2014 247 951
- US-A1- 2017 112 671

## Description

### RELATED APPLICATIONS

This application is related to and claims the benefit of U.S. Provisional Application 62/679,982, filed June 04, 2018, and entitled "Hearing Protection and Noise Recording Systems and Methods," and U.S. Provisional Application 62/856,070, filed June 02, 2019, and entitled "Hearing Protection and Noise Recording Systems and Methods,".

### GOVERNMENT RIGHTS

This invention was made in part under contract with the United States Army Research Office, under Contract No. W81XWH18C0044, and the United States Government may have certain rights in the invention.

### FIELD

The present invention relates to systems and methods for enhanced hearing protection and noise recording for auditory system health monitoring and compliance purposes. In particular, the systems and methods mitigate hearing loss in humans and animals such as military working dogs.

### BACKGROUND

The most common service-connected disability for military veterans is hearing loss. The CDC analyzed data from the 2010 Annual Social and Economic Supplement to the Current Population Survey and found that severe hearing impairment (SHI) was higher in veterans than nonveterans. Veterans were 30% more likely to have SHI than nonveterans after adjusting for age and current occupation. Further, veterans who served during September 2001 to March 2010, the era of overseas contingency operations (including Operations Enduring Freedom and Iraqi Freedom), were four times more likely than nonveterans to have SHI. The U.S. military has recognized the impact of Noise Induced Hearing Loss (NIHL) and tinnitus on the overall health of service members and veterans. Tinnitus and NIHL represent the largest number of VA claims for service related disability, affecting over 2.5 million veterans at a cost of over $1 billion per year. NIHL is characterized by temporary noise-induced shifts in hearing thresholds with recovery trajectories on the order of weeks. While cochlear hair cells in the auditory system survive temporary NIHL, there is an irreversible loss of cochlear neuron synapses in acute temporary NIHL, followed by progressive degeneration of cochlear neurons, even when cochlear hair cells recover normal function following temporary NIHL. It is therefore critical to quantify and characterize both environmental noise exposure as well as neurophysiological responses to auditory stimuli in order to characterize, assess, and mitigate negative effects of chronic noise exposure.

Furthermore, recent studies have shown that temporary threshold shifts may cause delayed degeneration of the cochlear nerve, contributing to NIHL and tinnitus beyond that identified using conventional threshold testing. To improve hearing protection, the Army has deployed Tactical Communication and Protection Systems (TCAPS). TCAPS combines a conventional earplug with a communication headset that can interface with military radios or pass through sound via a microphone that is located external to the headset. TCAPS adaptively amplifies signals and prevents pass through of hazardous sound levels. In quiet environments, the soldier is able to hear low level sounds needed to maintain situational awareness. In higher noise environments, the acoustic signal is attenuated to block hazardous sounds. However, neither TCAPS nor any of other protective devices issued by the U.S. Military provide any capability to determine compliance with standard operating procedures for proper use of the device. Furthermore, existing systems cannot collect auditory data for examining system performance or for predicting hearing loss patterns.

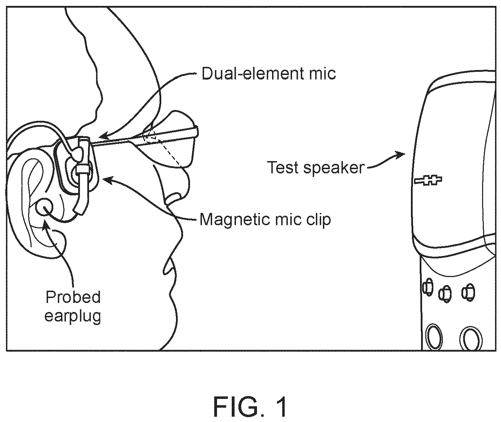

The effectiveness of any hearing protection device is impacted by (1) the user's compliance (whether they use hearing protection when required); and (2) the training of the user (whether they wear it consistently and properly). Assessment of hearing protection as specified in ANSI S12.6 has moved from single measurements to measurement across multiple users for a more realistic assessment of device performance. However, these measurements do not fully account for individual variations due to fit and are not useful outside a laboratory setting. A Field Microphone-In-Real-Ear (F-MIRE) system, shown in FIG. **1**, uses a dual element microphone to measure sound levels in the ear canal for comparison to sound levels outside of the earplug. While an effective compliment to Real Ear at Threshold (REAT) measurements, there are several challenges to this approach which include the need to prevent increased levels of insertion loss and lack of a form factor compatible with "non-laboratory" usage. Further, the system does not have the capability to record and stored sound level data that may be downloaded to a suitable device, e.g., personal computer, smart mobile device, and the like, for further analyses.

Military working dogs (MWDs) are similarly vulnerable to suffer from SHI due to the auditory assault caused during combat and non-combat training. MWD training has been reported to cost $80,000 to $100,000 per dog. NIHL has also been reported as an issue for hunting dogs and MWDs. As in humans, the main mechanism of NIHL is hair cell damage, but extreme noise can also rupture the tympanic membrane or cause damage to the ossicles. In addition, the noise level in kennels can cause cumulative damage. Despite well documented evidence that noise in a military environment can damage a working dog's hearing and degrade its performance, limited options are available for hearing protection. While over-ear solutions (e.g. Mutt Muffs) provide some level of protection for high noise environments (e.g. aircraft, auto racing, concerts), their effectiveness and utility in a military environment is limited. Military working dogs spend significant time in high noise environments, exposing them to significantly higher instantaneous and average noise levels, which can result in acute and chronic hearing degradation. In addition, it is essential that the dog's handler be able to communicate with the working dog in high noise environments. Passive hearing protection does not discriminate between desired and undesired sound and effectively disrupts any communication with the handler. Although hand or other visual signals may help, in many situations they are of only limited applicability. Further, over-ear or muff-type protection provide less than optimal protection against hearing loss due to the difficulty in maintaining a reliable seal between the muff and the area around the dog's ear. The combination of animal fur and physical stresses on the muff greatly reduces the reliability of the seal.

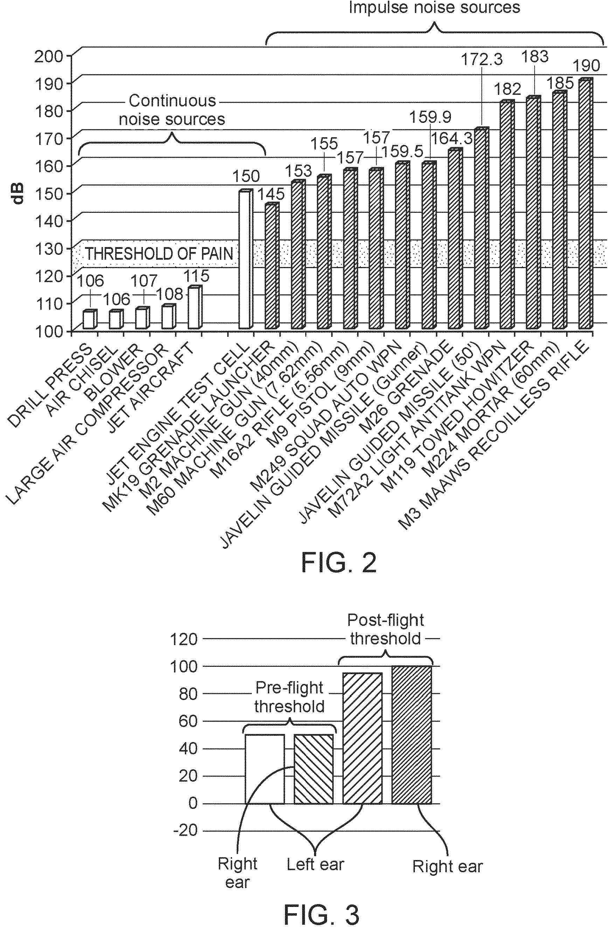

Noise affecting MWDs in military environment is caused by a variety of sources that includes impact or impulse noise from weapons or explosive devices, broadband noise from machinery such as turbines and jet engines, tonal noise from rotating machinery such as helicopter blades and internal combustion engines, vocalization by humans (including but not limited to speech), and kennel noise. All of these sources contribute to overall noise levels and lead to hearing loss and create challenges for the handler. FIG. 2 displays sound levels (dBA) for broadband sources and impulse sources (dBP) for some noises present in military operations. The measured noise levels in the cockpit of Army helicopters is reported to be at least 106 dBA, and sound levels of at least 122 dB SPL (sound pressure level) in an operating military helicopter has been reported. Helicopters generate lower frequency sound spectra (below~ 1000 Hz), typically dominated by tonal components. The effects of this noise level in a short 30-minute helicopter flight is illustrated in FIG 3. As shown, a Temporary Threshold Shift (TTS) of approximately 50 dB SPL is induced by exposure to noise. It is likely that upon exiting the aircraft, normal vocal commands will not be heard or will be misinterpreted by the dog at a critical juncture in its mission. The maximum human hearing range is between about 31Hz and about 19kHz while the canine hearing range is between about 64Hz and about 44kHz.

Hearing protection devices (HPD) are traditionally divided into two categories, namely, linear devices in which sound attenuation is constant and does not depend on the external sound level, and non-linear devices in which attenuation is a function of the amplitude of the sound level. Non-linear devices are generally of two varieties, namely, (1) passive devices such as earplugs or headset (ear coverings) which utilize mechanical or electrical means, such as the Single-Ended Combat Arms Military Ear Plugs (The 3M Company) and Peltor^{™} Tactical Earplug (the 3M Company) to pass low level sounds with minimal attenuation and selectively block loud continuous or impulse noise as external noise levels increases and (2) active noise cancellation (ANC) systems in which sound is measured with a reference microphone and filtered to generate a canceling noise signal. Non-linear earplugs or headsets usually include a sound path with acoustic impedance; for example, they may comprise of a cylindrical element perforated at either end, which is inserted into an earplug. Alternately, they may use an external microphone with noise played back at an appropriately reduced level via a miniature loudspeaker placed inside the HPD close to the listener's ear. In ANC systems, the canceling signal is typically generated using a small speaker or acoustic receiver. Active noise control systems work best at lower frequencies and are most effective when combined with effective passive attenuation. Non-linear devices protect the ear against loud, impulsive noise while allowing an almost unaltered perception of faint or moderate level sounds such as oral communication.

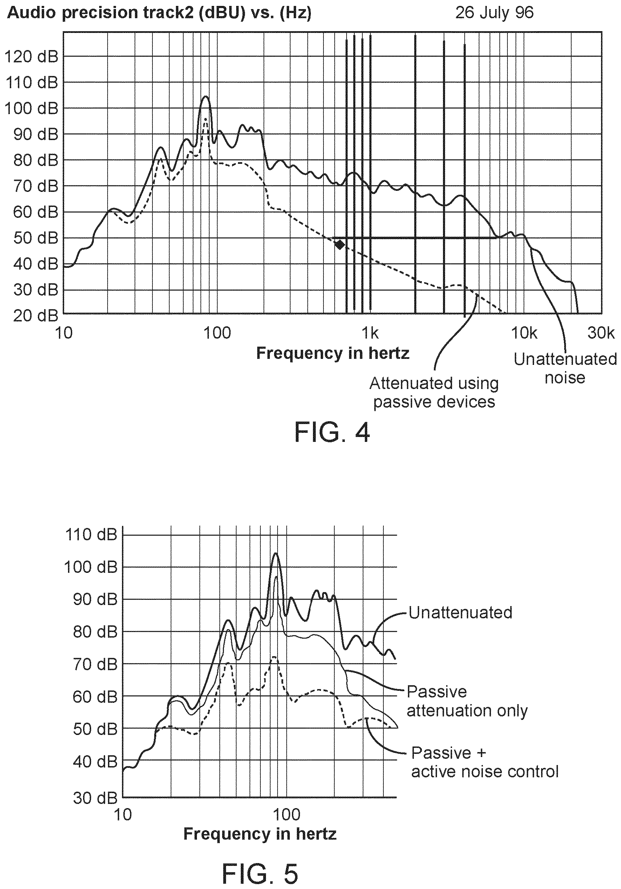

Broadband noise can be produced by a variety of sources including impulsive sources (e.g. gunshot or explosive) or machinery. Both have significant noise components with frequency in the range of 10 Hz to 10 kHz overlapping the range of human speech (approximately about 300 Hz to about 3400 Hz) and interfering with voice communications. On the other hand, aircraft with rotating blades (helicopters, turbo props) generate lower frequency spectra, typically dominated by tonal components. Passive earplugs are very effective in reducing noise above 200 Hz but have little effect at lower frequencies (FIG. 4), thereby limiting the effectiveness of the passive system. Addition of Active Noise Control (ANC) can significantly improve performance (FIG. 5) at low frequencies but produce minimal additional attenuation above 200 Hz.

U.S. Pat. No. 9,628,895 entitled "Animal Headphone Apparatus," describes a modified headphone device specifically suited for use with dogs so that an owner may allow their dog or other pets to listen to music. The owner may also use the headphones to command, train or monitor their pet. A first earpiece and a second earpiece are oriented inward and downward to account for the substantially vertical structure of a canine ear canal. A chipset and a power source provide capability for several features, including an integrated music player, at least one camera, a microphone, a vibration producing device, a global positioning system device, and at least one digital display for video viewing by the dog or other pet. A wireless communication device allows a user to control various features through a smart phone or remote control. This device is a headset which allows the owner to play music or speak to the dog and is intended to help soothe and calm the dog without requiring the owner to listen to the same music. The system is not intended to protect the dog's hearing from loud noises.

Systems and methods for protecting against hearing loss and capable of recording sound level data for analysis and diagnosis of hearing loss are needed.

### BRIEF DISCLOSURE

Disclosed is an exemplary hearing protection and noise recording system (HPRS), not forming part of the invention, comprising a noise reduction element to attenuate external noise to a predetermined in-ear threshold noise level, a coder-decoder element comprising one or more analog to digital converters to digitize one or more signals from the noise reduction element, a controller capable of bidirectional communication with the coder-decoder element for proceesing output from the coder-decoder element and for controlling the operation of the hearing protection and recording system, and a recording element capable of bidirectional communication with the controller to record output from the controller as a function of time. The noise reduction element may comprise at least one of a linear noise reduction device and a non-linear noise reduction device. The non-linear noise reduction device may comprise at least one of passive earplugs capable of increasing attenuation of external noise as the external noise level increases and an active noise cancellation subsystem. The predetermined in-ear threshold noise level is a noise level that is lower than the external noise level by up to 30 dB. The passive earplugs may be capable of attenuating external noise at frequencies of at least about 200 Hz. The active noise cancellation subsystem may be capable of attenuating external noise at frequencies between about 200 Hz and 1000 Hz. The noise reduction element may comprise at least one microphone to external noise and at least one microphone to capture in-ear noise. The system may further comprise at least one sensor element in communication with the coder-decoder element and the controller to enable correlation of noise reduction with physiological parameters. The sensor element may comprise at least one of an accelerometer, an acoustic source, a temperature sensor, an optical sensor, and a pulse oximetry sensor. The system may further comprise an acoustic source capable of being triggered by the controller to generate a noise signal for measuring acoustic impedence of the ear. The acoustic source may be a miniature speaker. The system may further comprise a communication element operably connected to the controller. The communication element may be configured to provide bidirectional communication between the user of the HPRS system and a third party. The system may further comprise one or more rechargeable batteries and an inductive coil for charging the batteries using an external induction battery charger.

Disclosed is an exemplary hearing protection and noise recording system (HPRS), not forming part of the invention, comprising a noise reduction element to attenuate external noise to a predetermined in-ear threshold noise level, a plurality of comparators for sampling external noise and in-ear noise generated by the noise reduction element and operably connected to a controller, and a recording element capable of bidirectional communication with the controller to record data output from the controller wherein the controller proceeses output from the plurality of comparators and controls the operation of the hearing protection and recording system. The noise reduction element may comprise at least one of a linear noise reduction device and non-linear noise reduction device. The non-linear noise reduction device may comprise at least one of passive earplugs capable of increasing attenuation of external noise as the external noise level increases and an active noise cancellation subsystem. The predetermined in-ear threshold noise level is a noise level that is lower than the external noise level by up to 30 dB. The passive earplugs may be capable of attenuating external noise at frequencies of at least about 200 Hz. The active noise cancellation subsystem may be capable of attenuating external noise at frequencies between about 200 Hz and 1000 Hz. The system may further comprise at least one sensor element operably connected to a coder-decoder element and the controller to enable correlation of noise reduction with physiological parameters. The at least one sensor element may comprise one of an accelerometer, an acoustic source, a temperature sensor, an opticl sensor, and a pulse oximetry sensor. The system may further comprise an acoustic source operably connected to a coder-decoder element and the controller. The acoustic source may be capable of being triggered by the controller to generate a noise signal for measuring acoustic impedence of the ear. The system may further comprise a communication element operably connected to the controller. The communication element may be configured to provide bidirectional communication between the user of the HPRS system and a third party. The system may further comprise one or more rechargeable batteries and an inductive coil for charging the batteries using an external induction battery charger. The noise reduction element may comprise at least one microphone to capture external noise and at least one microphone to capture in-ear noise. The controller may comprise a flash memory element for storing captured data, at least one data transfer element for encrypting and transmitting captured data to an end device for analysis, and a plurality of I/O channels for hosting one or more sensor elements. The captured data may be transmitted to an end device at predetermined time intervals via at least one of WiFi connectivity and Bluetooth connectivity. The end device may comprise at least one of a smart phone, smart tablet, and a data server.

Disclosed is an exemplary method for hearing protection and noise recording, not forming part of the invention, using a hearing protection and noise recording system comprising collecting external noise and in-ear noise using one or more microphones, converting the signal output from the one or more microphones to one or more digital signals, processing the one or more digital signals using a controller, attenuating external noise levels to an in-ear threshold noise level, and recording external noise and in-ear noise data output from the controller as a function of time. The attenuating step may comprise at least one of passive noise attenuation and active noise attenuation. The converting step may be done using one or more comparators. The converting step may also be done using a coder-decoder element comprising one or more analog to digital converters. The in-ear threshold noise level is a noise level that is lower than the external noise level by up to 30 dB. The disclosed method may further comprise the step of comparing in-ear noise level data and external noise level data to determine whether the hearing protection and noise recording system is being used in compliance with standard operating procedures. The method may further comprise the step of monitoring neurological response of the auditory system to external noise using coherent fNIRS. The method may further comprise the step of correlating neurological response to hearing loss.

Disclosed is an exemplary hearing protection and communication system for a dog according to claim 1.

Other features and advantages of the present disclosure will be set forth, in part, in the descriptions which follow and the accompanying drawings, wherein the preferred aspects of the present disclosure are described and shown, and in part, will become apparent to those skilled in the art upon examination of the following detailed description taken in conjunction with the accompanying drawings or may be learned by practice of the present disclosure. The advantages of the present disclosure may be realized and attained by means of the instrumentalities and combinations particularly pointed out in the appendant claims.

### DRAWINGS

The foregoing aspects and many of the attendant advantages of this disclosure will become more readily appreciated as the same becomes better understood by reference to the following detailed description, when taken in conjunction with the accompanying drawings, wherein:
FIG. **1****.** An exemplary Field Microphone-in-Real-Ear (F-MIRE) system (prior art).
FIG. **2****.** Sound levels for military noise sources.
FIG. **3****.** Canine hearing threshold for left and right ears.
FIG. **4****.** Typical performance of passive noise reduction devices. Takeoff noise of a Cessna 172RG Cutlass airplane (top). Noise reduction by passive devices (bottom).
FIG. **5****.** Improved low frequency noise reduction. Unattenuated (top), passive noise reduction (middle) and attenuation using a combination of passive and with ANC system (bottom).
FIGS. **6**A and **6**B, not forming part of the current invention, depict a schematic diagram of an exemplary in-ear hearing protection, and recording system, and a schematic diagram of an exemplary low-power hearing protection and recording system, respectively.
FIGS. **7**A and **7**B depict an exemplary ANC canine hearing protection system and components, and a cross sectional drawing of an exemplary ANC canine hearing protection system, respectively.
FIGS. **8**A and **8**B shows perspective views of an exemplary hearing protection, and communication systems for canines worn over the ear and in-ear, respectively.
FIG. **9**. Schematic diagram of an active noise cancellation sub-system.
FIG. **10****.** Perspective view of over-the-ear device for canines with ear-muff and hard shell.
FIG. **11****.** Perspective view of an in-ear device for canines with a soft hood.

The failure to number an element in a figure is not intended to waive any rights. Unnumbered references may also be identified by alpha characters in the figures and appendices.

The following detailed description includes references to the accompanying drawings, which form a part of the detailed description. The drawings show, by way of illustration, specific embodiments in which the disclosed systems and methods may be practiced. These embodiments, which are to be understood as "examples" or "options," are described in enough detail to enable those skilled in the art to practice the present invention. The embodiments may be combined, other embodiments may be utilized or structural or logical changes may be made without departing from the scope of the invention. The following detailed description is, therefore, not to be taken in a limiting sense and the scope of the invention is defined by the appended claims

In this document, the terms "a" or "an" are used to include one or more than one, and the term "or" is used to refer to a nonexclusive "or" unless otherwise indicated. In addition, it is to be understood that the phraseology or terminology employed herein, and not otherwise defined, is for the purpose of description only and not of limitation. For construing the scope of the term "about," the error bounds associated with the values (dimensions, operating conditions etc.) disclosed is ± 10% of the values indicated in this disclosure. The word "substantially" used before a specific word includes the meanings "considerable in extent to that which is specified," and "largely but not wholly that which is specified." In addition, "sound" and "noise" may be interchangeably used.

### DETAILED DISCLOSURE

Particular aspects of the invention are described below in considerable detail for the purpose for illustrating its principles and operation. However, various modifications may be made, and the scope of the invention is not limited to the exemplary aspects described.

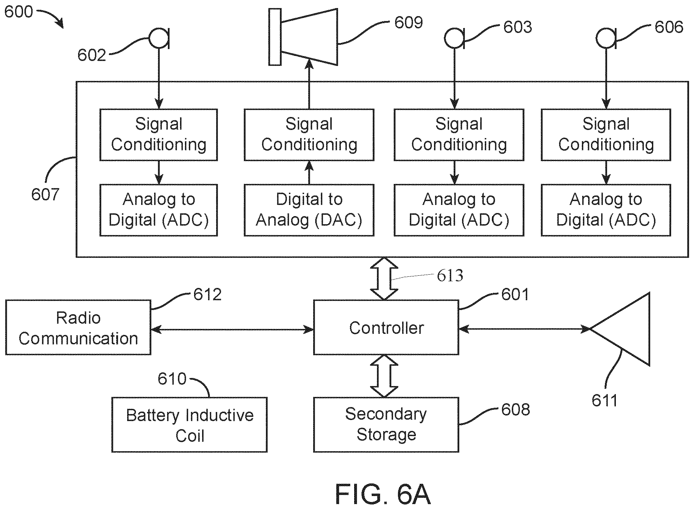

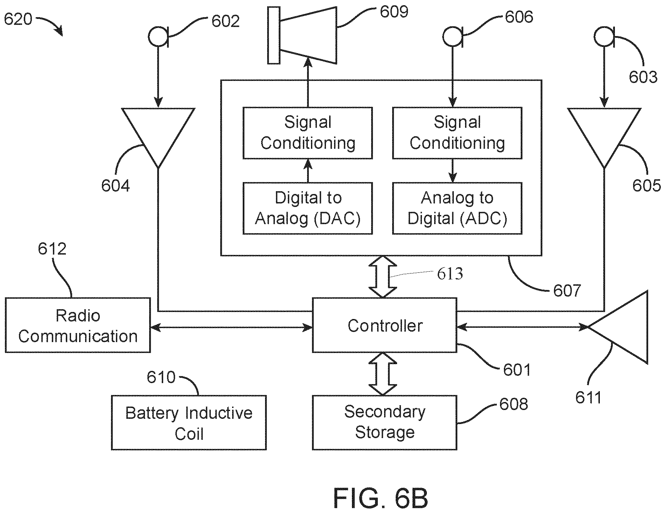

A schematic diagram of an exemplary in-ear hearing protection and recording system **600** ("HPRS"), not forming part of the current invention, is shown in FIG **6**A. System **600** may be controlled using controller **601.** An exemplary controller **601** may comprise the Bluetooth 4.2 enabled BGM121 microcontroller manufactured by Silicon Labs. Controller **601** is preferably 6.5 x 6.5 x 1.4 mm in size and has the capability to run end-user applications on-board or alternatively used as a network co-processor over one of the host interfaces. Other controllers may comprise Arduino or STM32 microcontrollers or possibly more capable microprocessor systems with multiple cores such as a Raspberry Pi controller, and could also be utilized at the expense of power and size. Controller **601** provides for multiple analog/digital inputs and outputs that enable integration of additional sensing capabilities using a plurality of sensors **606** to correlate at least one of hearing capability, noise levels, and noise reduction with physiological parameters and to improve the performance of the HPRS system. Controller **601** is a System-on-Chip (SoC) that has an integrated radio that supports hardware encryption. Controller **601** communicates with CODEC **607** via a serial bus, preferably with a resolution of at least 12 bits and at atleast about 44 K samples/second. A codec may encode a data stream or a signal for transmission or storage, possibly in encrypted form, and also decode the encoding for playback or editing. A CODEC such as the Analog Devices ADAU1772 or ADAU1777 may be employed to digitize multiple analog microphone signals from external microphone **602** and internal microphone **603** and stream data to and from serial ports on controller **601.** While increasing power consumption, this capability combined with its Pulse Width Modulation output enables data collection to support analysis of the acoustic response of the ear. Internal (in-ear) microphone **603** may be placed either in the ear plug or inside a headset to measure a signal which is representative of the noise exposure level inside of the hearing protection device). Ouput signals/information from the microphones may be processed by a controller module, captured/stored, and may be used to drive a speaker to transmit desired sounds and/or a noise canceling signal. The controller module may either be embedded in the earplug or reside in an external wearable module which can be attached to clothing and communicate with the earplug via a cable or wireless means. System **600** may include at least 16MB flash memory for secondary storage **608.** Stored data may be encrypted prior to transmission via Bluetooth or wireless communication to a smart phone or other devices for data capture and analysis. The flash secondary storage provides the system over a 24-hour period with a 194 byte per second data density, which can be increased by reducing the frequency at which the data is logged. In addition, a 32MB memory may be used if a larger form factor may be accommodated. Finally, controller **601** may generate a control signal to be used to drive an acoustic source **609** such a Peerless 50 mm headphone speaker using the digital CODEC **607.** Controller **601** may comprise embedded analog-to-digital and/or digital-to-analog capability, in which case, an external CODEC is not required. Digital devices such as an Arduino or STM32 Microcontroller may be operably connected to CODEC **607** via a digital data bus **613** to provide bidirectional communication and general purpose I/O channels may be used to interface with a variety of devices such as physiological sensors. Controller **601** output may include, but is not limited to, external noise and in-ear noise, and ouput of the at least one sensor element. which may be in the form of digitized output from the coder-decoder element. The controller may also extract stored data and use it to continuously improve the noise reduction capability of the system, for example, using predictive tools such as machine learning and artificial intelligence.

Additional I/O channels may be available on controller **601** to support one or more sensors **606.** For example, addition of an accelerometer may provide valuable insights into the user's activity (e.g. running, sitting, jumping) profile. Similar systems in cell phones have shown to accurately classify user activities. The digital outputs from one or more of these sensors may be converted to analog output using converter **607,** which may be an on-chip peripheral of controller **601.** For example, an acoustic source **609** (when triggered by codec 607) may be used for measurement of acoustic power flow or impedance. With inclusion of a miniature acoustic source **609,** system **600** may provide measurement of auditory function (e.g., otoacoustics emission or middle ear impedance). The acoustic source may be used to identify unusual conditions such as ear drum perforations and capture information related to health of the tympanic membrane and external auditory meatus. A miniature deadphone speaker for example may be used to drive a known signal and amplitude and phase changes may be calculated to measure acoustic impedance. Measurement of acoustic power flow and normalized impedance may help to identify unusual conditions such as eardrum perforations. The acoustic source may also be used to capture information related to the functionality of the tympanic membrane, external auditory meatus (outer ear canal), and the ossicular chain. In another exemplary aspect of system **600,** sensors that measure temperature and pulse co-oximetry may be added to correlate the outputs of these sensors to performance of the system and auditory health of the user. Other sensors such as a GPS sensor and a camera may also be used to provide position location information. Pulse oximetry non-invasively measures the oxygen saturation (SaO₂) of hemoglobin in arterial blood or the average amount of oxygen bound to each hemoglobin molecule. Optical or electrode sensors may also be use to measure Brainstem Evoked Auditory Response as a way of measuring the response of the auditory system to environmental noise. Data (e.g. noise levels, sensor outputs) in the form of raw data or processed data output from the controller **601** may be recorded/stored in storage device **608.** System **600** may also use utilize two or more microphones to continuously capture and record external noise and in-ear noise measurements and to enable noise reduction using the non-linear passive and active noise reduction methods disclosed herein.

In another exemplary HPRS **620 (****FIG. 6**B), not forming part of the current invention, the analog outputs of external microphone **602** and internal microphone **603** may be sampled by low power comparators **604** and **605** respectively. A comparator may convert the analog voltage signal from a microphone to digital output. These comparators would replace CODEC **607.** The Silicon Labs BGM121 is an example of processor that has been optimized for low power (approximately less than 100 mW). A low power HPRS may be used when its primary use is to monitor and reduce noise levels. The comparators are preferably on-chip peripherals of controller **601** and may sample at either 8 or 16 bits in low or medium power mode. Preferably, controller **601** has the capability to stay in sleep mode while the comparators **604** and **605** are accumulating data, which extends the life of one or more rechargeable batteries (not shown in FIG. **6**B) that powers system **620.** In the low power mode, system **620** may operate for several days between charging of the battery power supply. System **620** may be able to sample and store data over several days by collecting data at a low sample rate. This data may be used to verify compliance related use of the system, and provide a time record of both continuous and impulse noise. Advanced analysis of the acoustic data collected by the auditory system may also be supported. The system is capable of sampling and generating signals at audio rates (typically 44.1kHz) to support periodic intervals of data collection. As previously described, system **620** may have multiple general purpose I/O lines to support the addition of other sensors (e.g. acceleration and/or heat) and also drive sound sources for acoustic characterization of the auditory system. These alternate options may require higher power consumption which may limit use to several hours before recharging. Addition of a behind-the-ear unit (similar to a hearing aid) could host at least one of additional sensors, memory and power modules. A behind-the-ear unit would not interfere with use of standard headphones or other gear typically worn by the warfightermilitary or law enforcement.

System **600** or **620** may be completely sealed and wire (cord) free; that is, one or more batteries in system **600** may be recharged wirelessly inductive coil **610** that may be energized using an external inductive battery charger. Data (microphone outputs, sensor outputs) may be processed by controller **601** and recorded and stored in temporary local memory in system **600** or **620** and recorded data may be downloaded to one or more secondary storage devices **608.** Examples of storage device **608,** include, but is not limited to, flash memory cards such as secured digital (SD) cards and micro SD cards and storage drives such as solid state drives (SSD). Alternately, storage **608** may be located in mobile devices such as smart phones, tablets, and the like, which are configured to communicate with system **600** or **620** using wireless protocols such as Bluetooth. Alternately, recorded data from the system **600** or **620** may be downloaded to one or remote server via the internet. One or more mobile devices may access downloaded data using wired or wireless communication methods. A suitable application program ("app") operable on the one or more mobile devices may be used to authenticate and permit access and communication between the one or more mobile devices and one or more remote server. System **600** may maintain a running log of timestamped external and internal sound levels and the time of exposure.

Impulse noise levels from small arms, artillery and mortar fire can exceed 180dB, which is well above the dynamic range and peak noise levels of between about 120dB to about 130dB of available miniature microphones. To enable the use of miniature microphones, nonlinear components may be used to compress high noise levels. Inner ear measurement may be extrapolated to infer impulse peak levels, and passive materials may be used to compress sound levels.

System **600** may further comprise at least one communication element **612** (e.g. microphone, radio system) operably connected to controller **601** and configured to provide bidirectional communication between the user of the HPRS system and a third party.

In an exemplary in-ear recording and hearing protection system, an optical neural sensor component may be used in addition to system **600.** The optical neural component may comprise a classical electroencephalogram (EEG) sensor, or a functional near-infrared spectroscopy (fNIRS) system, in a wearable form factor (similar to a behind-the-ear hearing aid or headset mounted device) that may record and timestamp neurological response of the auditory system to environmental (external) noise and enable identification of any deterioration in hearing. Traditional audiological methods assess a range of neurophysiological responses to auditory stimuli using electrophysiological and psychoacoustic tests and have limited utility outside a controlled laboratory testing environment. Classic electrophysiological methods typically include auditory brainstem responses (ABRs) elicited by pure tones and complex stimuli, the frequency following response (FFR) elicited by voiced speech and complex auditory stimuli, and optoacoustic emissions (DPOAEs) to assess cochlear function, while psychoacoustic tests probe hearing detection thresholds. While these tests provide well-characterized measures for quantifying neural responses elicited by controlled stimuli, they are challenging to implement in field-deployable devices because of artifacts generated by muscles, movement, and environmental noise. Recent advances by the Johns Hopkins University Applied Physics Laboratory (JHU/APL) in non-invasive Brain Computer Interfaces offer the possibility to better characterize neural activity in the brain through optical neural imaging. Since fNIRS uses simple near-IR lasers, it can readily be miniaturized to be compatible with a wearable format.

fNIRS is a noninvasive functional neuro-imaging method that tracks changes in signal propagation of diffuse light sources at near-infrared wavelengths through biological tissue. fNIRS can monitor hemodynamic activity in the brain by tracking absorption spectra of oxygenated and de-oxygenated hemoglobin. The use of fNIR and fMRI methods as functional imaging methods relies on the principle of neuro-vascular coupling also known as the hemodynamic response or blood-oxygen-level dependent (BOLD) response. Through neuro-vascular coupling, neuron activity is linked to related changes in localized cerebral blood flow. fNIR has several advantages in cost and portability over fMRI. The spatial resolution of fNIRS is on the order of about 1 cm with up to 1.5 cm signal penetration depth. Skin, tissue, and bone are mostly transparent to NIR light in the spectrum of 700 nm to 900 nm, while hemoglobin (Hb) and deoxygenated-hemoglobin (deoxy-Hb) are stronger absorbers of light. Differences in the absorption spectra of deoxy-Hb and oxy-Hb allow the measurement of relative changes in hemoglobin concentration through the use of light attenuation at multiple wavelengths. Two or more wavelengths are selected, with one wavelength above and one below the isosbestic point of 810 nm at which deoxy-Hb and oxy-Hb have identical absorption coefficients. Using the modified Beer-Lambert law (mBLL), relative concentration can be calculated as a function of total photon path length. Typically, the light emitter and detector are placed ipsilaterally on the subject's skull such that recorded measurements are due to back-scattered (reflected) light following elliptical pathways. Studies relating fMRI (functional magnetic resonance imaging) and fNIR show highly correlated results in cognitive tasks. Multiplexing fNIRS channels may allow 2D topographic functional maps of brain activity (e.g. with Hitachi ETG-4000 or Artinis Oxymon) while using multiple emitter spacings may be used to build 3D tomographic maps. While resolution on the order of centimeters is considered low compared to functional magnetic resonance imaging (fMRI), it is well suited for integrating over auditory cortex with a low number of channels.

Recently, JHU/APL have utilized coherent processing methods to improve the resolution (3-5mm) of fNIRS to approach that of fMRI increasing the potential utility of fNIRS. A coherent fNIRS sensor may be compactly integrated into a comprehensive solution for monitoring noise exposure and auditory function using a combination of in-ear sources and headband-located detectors for targeting the auditory cortex. In combination with auditory monitoring methods used in neo-natal intensive care units (NICU) environments, this system may provide data to support development of models characterizing the complex relationship between environmental noise exposure and hearing injury. Improved understanding of the relationship between noise exposure and auditory processing would, in turn, allow us to better protect hearing with user-friendly, comfortable devices and interventions. In contrast to electrophysiological and psychoacoustic auditory tests, fNIRS is ideally suited for measuring cortical and metabolic responses to environmental auditory stimuli in a field-deployable wearable device. A fNIRS system is less sensitive to motion and muscle artifacts than traditional electro-physiological sensors and can be packaged into a wearable mobile device.

In the exemplary systems disclosed above, hearing protection, that is, noise attenuation, may be achieved by using passive and active noise reduction components and methods. The entire disclosures of U.S. Pat. No. 9,654,854 entitled "In-ear device incorporating active noise reduction," U.S. Pat. No. 6,278,786 entitled "Active noise cancellation aircraft headset system," U.S. Pat. Pub. No. 2015/0294662 entitled "Selective Noise-Cancelling Earphone," U.S. Pat. Pub. No. 2012/0014532 entitled "Noise Canceling Headphone," U.S. Pat. No. 7,058,368 entitled "Adaptive feedforward noise cancellation circuit," U.S. Pat. Pub. No. 2002/0141599 entitle "Active noise canceling headset and devices with selective noise suppression,".

Compliance with standard operating protocols may be determined using several factors. For example, the HPRS is typically expected to reduce external noise by about 20 dBA to about 30 dBA ("target attenuation"); however, due to variations associated with in fit of the device in or on the ear, the actual reduction may be somewhat lower. If the collected data for a particular user or system shows that the difference between the external noise level and in-ear noise level is less than the target attenuation, the data suggests a non-compliance event and corrective action may be taken. In addition, data from optional sensors such as body temperature sensors and ambient temperature sensors may also be examined to determine whether the HPRS was being worn by the user during a non-compliance event. A suitable decision matrix may be developed and implemented to determine user compliance with standard operating protocols of the HPRS. Non-compliance data may be used during assessment of any claims for damages brought by a user who asserts loss of hearing due to work environment. In addition, collected data profiles may be used to predict hearing loss of the user also.

Wearing/not wearing a HPRS by a user may also be determined using protocols that require monitoring of compliance related to wearing of the HPRS. Signals from a radio frequency unit in the HPRS may be connected by land line/mobile phone to a receiving base unit in a fixed location (e.g. workplace or third party monitoring location) and transmitted to a third-party service center who may monitors wearing/not wearing, tampering, breaking etc. GPS type monitoring may also be used.

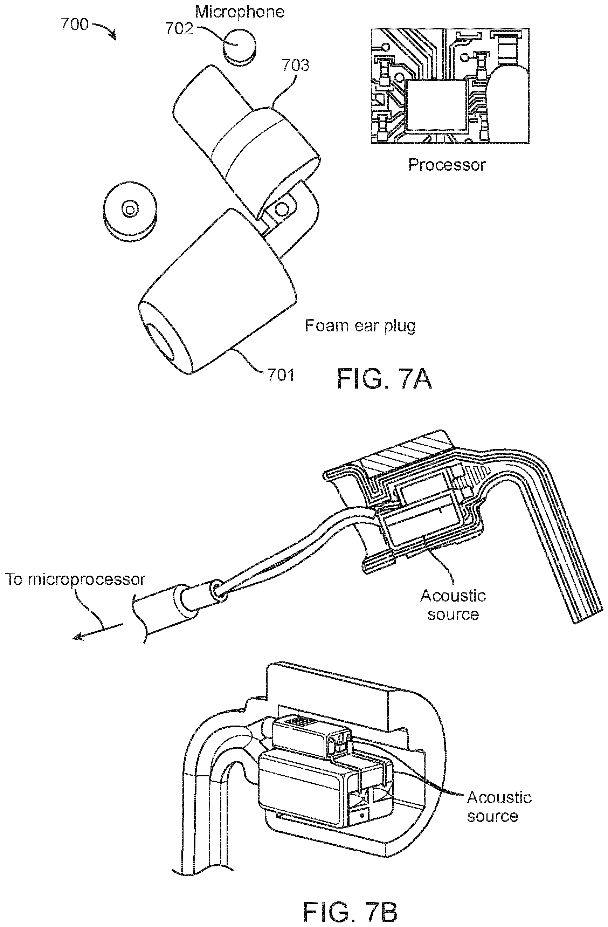

FIG. **7** shows an exemplary in-ear noise reduction (IER) system **700** designed for canines. System **700** includes a removable foam insert or ear phone tip **701** (e.g. Comply^{™} insert sold by Hearing Components, Inc.). Microphone **702** may be integrated into ear plug **701.** Housing **703** may be 3D printed, molded or machined and houses the sensor components such as an acoustic power source.

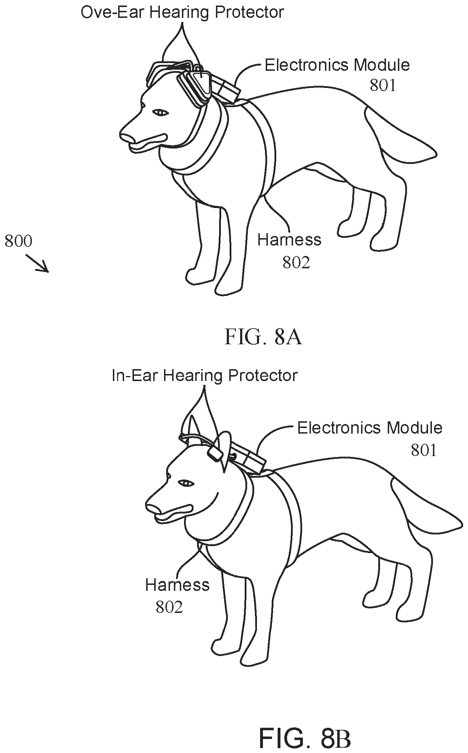

In an exemplary hearing protection, and communication system for animals such as military working dogs (MWD), the system may be designed to fit in-ear or over-the-ear (FIG. **8****).** The system comprises a noise reduction element to attenuate external noise to a predetermined in-ear threshold noise level, a plurality of comparators for sampling external noise and in-ear noise data generated by the noise reduction element and operably connected to a controller wherein the controller controls the operation of the hearing protection system, and a communication element opearably connected to the controller and configured to enable the dog's handler to communicate with the dog. The communication element comprises a bypass element to enable the handler to bypass noise reduction and transmit commands to the dog at frequencies greater than about 10 kHz, the hearing frequency range of dogs. Commands may be preferably transmitted at frequencies greater than 20 kHz. At these frequencies, ambient noise levels are lower. Further, at these frequencies, sound is inaudible or minimally audible to the human ear. Commands may be processed using speech filtering methods to eliminate background noise. The noise reduction element comprises a non-linear passive noise reduction element capable of increasing attenuation of external noise as the external noise level increases and an active noise cancellation subsystem. The communication element may comprise at least one of a radio, radio receiver, a Bluetooth communication device, a wireless communication device, an antenna, and a user interface unit. The system may further comprise a recording element configured to record output from the controller. The communication element may be further configured to unencrypt commands at the dog's ear to the audible frequency range of between about 100 Hz and about 4 kHz. The exemplary system may comprise an in-ear system. The system may comprise an over-the-ear system.

Further, the system provides for communication between the handler and the MWD. A combination of passive and active noise cancellation technologies may provide noise attenuation across the entire acoustic spectrum and reach the 20 dB to 30 dB of noise reduction target across the canine hearing range. In addition to the recording aspects previously disclosed, an exemplary hearing protection, recording and communication system for MWD's may comprise at least one or more of the following components or capabilities:

### (A) PASSIVE NOISE REDUCTION ELEMENT FOR HIGH FREQUENCY NOISE ATTENUATION

The performance of passive noise attenuation components relies primarily on two factors, namely (1) selection of materials that effectively attenuate sound wherein a significant trade-off is made between weight of the material and performance, in particular, at lower frequencies. Heavy, ridged materials provide the best attenuation but increase overall weight. For this reason, typical passive systems are designed with a hard, plastic shell lined with lightweight acoustic foam, and (2) maintaining an effective seal between the headset (or earplug) and the dog's body. For over-the ear designs, FIG. **8**A, a mesh webbing or fabric sieve that holds the earmuff in place and maintains positive pressure is essential. The primary components of the passive hearing component for the over-ear prototype are a conformable outer shell, internal foam backing and material for creating a seal between the headset and dog's head. The size and shape of the dog's outer ear and the need to maintain a good seal against fur may require at least one of using deeper, more compliant, foam components, modifying the material that covers the foam to better couple through the fur and onto the skin, and, the potential for using a modified ultrasound transmission gel that would provide a better seal, without irritating the MWD. Exemplary gel materials include viscous, low volatility, gel materials that would enhance the seal between the compliant foam and the dog's skin.

For in-ear configurations, FIG. **8**B, preferably materials that expand to fill the cavity of the ear are employed to maintain a seal. The canine ear canal has a vertical and horizontal region and is L-shaped. The in-ear foam earplug should preferably be designed not to extend into the junction region between vertical and horizontal canal because it may be quite painful to the dog and can impact its hearing ability. The outer ear includes the pinna, which is designed to funnel sound to the ear canal. The pinnae are mobile, and a dog has much greater mobility of the outer ear compared with humans. Due to these structural differences, dogs have greater sound sensitivity over a broad frequency range compared to humans. In FIG. **8****,** the signal processor, radio receiver, and power supply **801** is held in place using a collar **802.** Alternatively, the electronics module can be located on a vest worn by the dog.

### (B) ACTIVE NOISE CANCELLING (ANC) ELEMENT

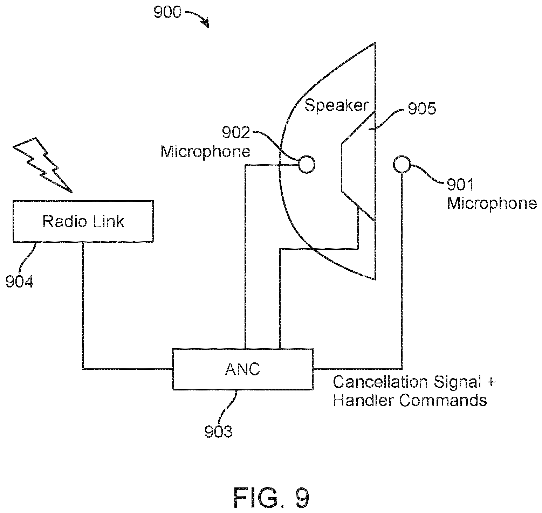

This element (or sub-system) comprises: (1) a feedback microphone component to sense noise levels at the ear for anti-noise generation, which is preferably optimized for a dog's hearing range. Adaptive digital processing or analog processing algorithms are comparable to that used in ANC systems for human use, and (2) a feedforward component to sense loud impulse noise and shut off ANC. When the ANC is turned off, noise attenuation is by passive mode. An adaptive ANC system will typically utilize both the feedforward and feedback microphone in generating the anti-noise signal. Unlike ANC systems suitable for human use, in canine (e.g. MWD) hearing protection systems, the feedforward signal can consist of components that are out of the range of normal human hearing and allows a handler to communicate acoustically with the dog (e.g. use a dog whistle) without compromising the effectiveness of the noise reduction. Alternately, pre-recorded commands, for example, a command that makes the dog to sit and stay still, may be transmitted to the animal triggered by shut-off of the ANC sub-system or component due to an impulse noise. Additionally, the system can utilize compression methods to lessen the overall gain as noise levels increase. FIG. **9** is a schematic diagram of an exemplary ANC sub-system **900.** A residual noise microphone **901** is used to measure noise levels representative of levels at the tympanic membrane in the ear. The feedforward microphone **902** senses noise outside of the hearing protection device. The ANC controller **903** uses feedback and/or feedforward protocols to calculate an inverse signal proportional to the noise, which is sent to speaker **905.** Sound from the speaker (anti-noise) mixes with the noise to produce the noise reduction effect. For low frequency noise, the correlation time of the noise signal is significantly greater than the delay of the electronics and the transfer function is stable over time. Most commercially available ANC headsets utilize this approach, typically using analog electronics for low cost and power consumption. Adaptive filtering methods can be used to continuously account for variations in the systems response. Characteristics of the dog's ear may introduce complexities that may require adaptive digital signal processing. The feed forward microphone **902** also detects loud impulse noises and can stop operation of the ANC controller to prevent instabilities due to noise saturation. Variable gain may also be used to adjust the level on sound that is allowed to pass through the filter. Under these conditions when the ANC feature is disabled, noise attenuation may be achieved primarily by using the passive components of the hearing system. Further, the ANC controller may compress sound during intervals when the handler is communicating with the canine. As with the feedback system, this can be implemented with analog electronics. An ANC controller **903** that combines feed forward and feed-back modes may improve performance over only feedback mode and may require the use of a digital control system as previously described.

### (C) SHORT-RANGE COMMUNICATIONS SUB-SYSTEM

A miniature radio receiver **904** with a frequency ranges such as 225-450 MHz, 1250-1390 MHz, and 1750-1850 MHz may be built into the collar unit **801** and may communicate with a Rifleman Radio or alternately using frequencies compatible with Bluetooth or WiFi to another suitable communication device. Similarly, frequency range of 30 to 512 MHz may be used to communicate with the Multiband Inter/Intra Team Radio (MBITR). A simplified antenna may be built into the collar unit to enable short distance communication (approximately 50 meters) between the dog and the handler. The Rifleman Radio has 50 preset channels and 5 talk groups per preset, while the MBITR has 100 preset channels and menu selectable talk groups. In the exemplary hearing protection and recording system for dogs, for ease of use, the dog's receiver may be assigned to a single talk group comprising the handler and the dog to prevent the dog from becoming confused by other radio chatter.

### (D) USER INTERFACE/CONTROLLER

A user interface such as the Invisio V50 (made by Invisio Communications, Denmark) may be configured such that a set of push buttons may be preset to select voice or radio communication between the handler to the dog. A handler may assign the dog's talk group to an unused or less used push to talk (PTT) button on the user interface. for communication with the dog. Alternately, a simple single radio connection for the handler may be used with a PTT button to communicate with the dog. The interface is preferably capable of providing the following features: (a) integrate existing and covert operational communications systems to support stand-alone and unit operations; (b) capable of communicating with MWD and handler's group (humans), with communication with MWD assigned to separate PTT group; (c) bypass capability that allows the handler to bypass noise reduction to allow the handler to provide commands to the MWD. Features of bypass include: (1) compressing speech and mixing a radio signal into an active control signal or command. It may be difficult to filter undesired signal because an open microphone reduces communication effectiveness and increases noise levels, and (2) compressing and passing acoustic commands to the MWD at higher frequencies where noise levels are about 20 dB and 40 dB below background noise, and outside human hearing, which is less than about 20 kHz. Use of high frequency commands will not only improve the ability of the MWD to interpret commands in a noisy environment but also provide covert communication capability. Dogs can hear up to about 45 kHz (compared to about 20 kHz for humans). Most ambient noises roll off at about 10 kHz. By transmitting at higher frequencies, background noise is minimized, and the dog should be able to hear sounds that are out of the range of human hearing. Options for direct acoustic or radio communication will enable on-leash or off-leash and single handler or team operations.

### (E) OPTIONAL FEATURES OF SYSTEM 800

As previously described, real-time measurements of brain activity could be used to monitor hearing performance or measure the cognitive state of the dog. For example, a mapping of brain activity to external triggers may be used to provide a signal that allows a handler to determine whether the dog has detected something of interest, for example, like an explosive. The handler may also detect if the dog is getting tired or overloaded and needs rest. As an alternative to monitoring brain activity, the heart rate and body temperature (for example, inside the ear) of the dog may be measured and correlated to the dog's response to an external event (psychoacoustic responses) in instances where the dog's bark may not be heard by the handler when the dog is off-leash.

A critical requirement for effective noise dampening in canines is a tight seal between the ear and the device that allows the canine to tolerate the device to carry out its mission. In addition to providing hearing protection, the device also provides for communication between the handler and the canine (MWD) and this communication may be encrypted. In an exemplary over-the-ear device (ear muffs), sound dampening may be realized using both active and passive components as previously described. Communication may be encrypted such that a message sent from a handler device is transmitted at an acoustic frequency out of the range of human hearing and is unencrypted at the ear muff to the audible frequency range of speech of about 100 Hz and about 4 kHz. The frequency of human speech largely ranges between about 2 kHz and about 4 kHz. For MWD's, over-the-ear devices may be problematic due to one or more of the following reasons: attenuation of lower frequencies (100 Hz to 1000 Hz) may be difficult because of sealing issues, devices may be heavy and bulky and interfere with a typical military-style cage muzzle used on the MWD, and the encryption frequency may require the dog to be very close and in line-of-sight of the handler.

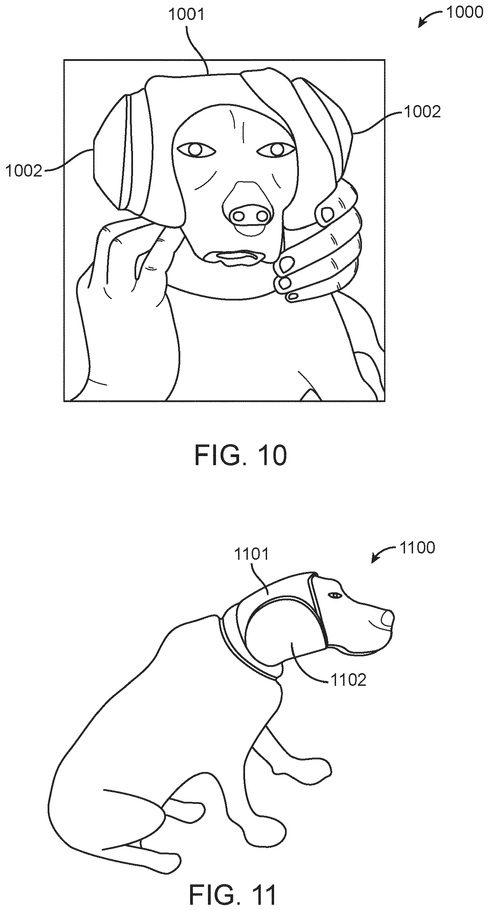

In another exemplary over-the-ear device (ear muffs) **1000,** sound dampening is realized using only passive components (FIG. **10****).** To obtain effective seals between the device and the ears of the MWD, a neoprene hood **1001** with hard shell muffs **1002** was used. Noise attenuation of the lower frequencies (100 Hz to 1000 Hz) was found to be ineffective. Hard ear muffs, for example, commercially available Mutt Muffs ^{™} and 4 Paws Aviation^{™} muffs do not stay on correctly to provide an effective seal when the system used in tactical situations, for example, when the dog is running.

In another exemplary over-the-ear device **1100** (FIG. **11**), the neoprene hood is retained for fit and compression, but the hard shells are replaced with acoustic foam sides to form a completely soft hood. Various commercial acoustic foams were tested and down selected to provide attenuation at low frequency (100 to 1000 Hz). Hood materials other than neoprene may be used to avoid over heating making the MWD uncomfortable.

In-ear devices (ear plugs) pose challenges related to MWD use. They may be hard to insert/fit for canines. Canine ear canals are different for different breeds and may require individual molds. They have the tendency to introduce dirt and bacteria into the ear canal. An exemplary in-ear passive device comprises a neoprene hood **1101** and acoustic foam ear pieces **1102** (FIG. **11****).** The ears may be outfitted with extra soft foam ear outlines. Noise attenuation of the lower frequencies (100 Hz to 1000 Hz) was found to be effective and ANC components are added to the device. Foam inserts comprise an acoustically, audiologically, and otology-safe foams that may be used to cover the internal electronics and provide a good fit in the canine ear canal. Individual ear molds may be used to fabricate inserts that anchor and stabilize the insert the ear canal of the dog. Communication components may also be integrated into this device.

Although the present disclosure has been described in connection with the preferred form of practicing it, those of ordinary skill in the art will understand that many modifications can be made thereto. Accordingly, it is not intended that the scope of the disclosure in any way be limited by the above description.

Further, the use of the transitional phrase "comprising" is used to maintain the "open-end" claims herein, according to traditional claim interpretation. Thus, unless the context requires otherwise, it should be understood that variations such as "comprises" or "comprising," are intended to imply the inclusion of a stated element or step or group of elements or steps, but not the exclusion of any other element or step or group of elements or steps. Such terms should be interpreted in their most expansive forms so as to afford the applicant the broadest coverage legally permissible.

## Claims

1. A hearing protection and communication system (800) for a dog, the system comprising:
a noise reduction element to sense and attenuate external noise to a predetermined in-ear threshold noise level;
a plurality of comparators (604, 605) for sampling external noise and in-ear noise data generated by the noise reduction element and operably connected to a controller (601) wherein the controller (601) is configured to control the operation of the hearing protection system; and,
a communication element (612) operably connected to the controller (601) and
configured to enable the dog's handler to communicate with the dog wherein the communication element (612) comprises a bypass element to enable the handler to bypass noise reduction and transmit commands to the dog at frequencies greater than about 10 kHz,
wherein the predetermined in-ear threshold noise level is a noise level that is lower than the external noise level by 20 dB to 30 dB, and
wherein the noise reduction element comprises at least one microphone (602, 603) to capture external noise and in-ear noise, a non-linear passive noise reduction element capable of increasing attenuation of external noise as the external noise level increases, and an active noise cancellation subsystem (900).

2. The system of claim 1 wherein the communication element (612) comprises at least one of a radio, radio receiver, a Bluetooth communication device, a wireless communication device, an antenna, and a user interface unit.

3. The system of claim 1 wherein the communication element (612) is further configured to decrypt encrypted commands at the dog's ear to the audible frequency range of between about 100Hz and about 4kHz.

4. The system of claim 1 wherein the system comprises an in-ear system.

5. The system of claim 1 wherein the system comprises an over-the-ear system.

6. The system of claim 1 wherein commands are transmitted at frequencies greater than about 20 kHz to minimize background noise.

7. The system of claim 2 wherein the communication element (612) is configured to transmit one or more pre-recorded commands to the dog when the active noise cancellation system (900) is turned off.

8. The system of claim 1 wherein the system is configured to turn off the active noise cancellation system (900) when loud impulse noises are detected by the at least one microphone (602, 603).

9. The system of claim 2 wherein the communication element is housed in an electronics module (801) configured to be strapped to the dog using a harness (802).

10. The system of claim 9 wherein the electronics module (801) further comprises a power supply.

11. The system of claim 10 wherein the radio receiver is configured to operate in selectable frequency ranges such as 30-512 MHz, 225-450 MHz, 1250-1390 MHz and 1750-1850 MHz to communicate with a suitable radio.

12. The system of claim 9 wherein the radio receiver is configured to communicate with at least one of a Bluetooth and WiFi enabled communication device.

13. The system of claim 1 further comprising at least one of a brain activity monitor, a heart rate sensor, and a body temperature sensor to correlate the dog's response to an external event.

## Patentansprüche

1. Ein Gehörschutz- und Kommunikationssystem (800) für einen Hund, wobei das System aufweist:
ein Geräuschreduzierungselement zum Erfassen und Dämpfen von Außengeräuschen auf einen vorbestimmten Schwellengeräuschpegel im Ohr;
eine Mehrzahl an Komparatoren (604, 605) zum Abtasten von Außengeräusch- und Im-Ohrgeräuschdaten, die von dem Geräuschreduzierungselement erzeugt werden, und die betriebsmäßig mit einem Steuergerät (601) verbunden sind, wobei das Steuergerät (601) ausgebildet ist, um den Betrieb des Gehörschutzsystems zu steuern; und,
ein Kommunikationselement (612), das betriebsmäßig mit dem Steuergerät (601) verbunden und so ausgebildet ist, dass es dem Hundeführer ermöglicht, mit dem Hund zu kommunizieren, wobei das Kommunikationselement (612) ein Bypass-Element umfasst, um es dem Hundeführer zu ermöglichen, die Geräuschreduzierung zu umgehen und dem Hund Befehle mit Frequenzen von mehr als etwa 10 kHz zu übertragen,
wobei der vorbestimmte Innenohr-Schwellenwertgeräuschpegel ein Geräuschpegel ist, der um 20 dB bis 30 dB niedriger ist als der Außengeräuschpegel, und
wobei das Geräuschreduzierungselement zumindest ein Mikrofon (602, 603) aufweist, zum Erfassen von Außengeräuschen und Im-Ohrgeräuschen, ein nichtlineares passives Geräuschreduzierungselement, das in der Lage ist, die Dämpfung von Außengeräuschen zu erhöhen, wenn der Außengeräuschpegel zunimmt, sowie ein aktives Geräuschreduzierungs-Teilsystem (900).

2. Das System nach Anspruch 1, wobei das Kommunikationselement (612) zumindest eines der folgenden Elemente umfasst: ein Funkgerät, einen Funkempfänger, eine Bluetooth-Kommunikationsvorrichtung, eine drahtlose Kommunikationsvorrichtung, eine Antenne und eine Benutzerschnittstelleneinheit.

3. Das System nach Anspruch 1, wobei das Kommunikationselement (612) ferner so ausgebildet ist, dass es verschlüsselte Befehle am Ohr des Hundes in einem hörbaren Frequenzbereich zwischen etwa 100 Hz und etwa 4 kHz entschlüsselt.

4. Das System nach Anspruch 1, wobei das System ein Im-Ohr-System umfasst.

5. Das System nach Anspruch 1, wobei das System ein Über-Ohr-System umfasst.

6. Das System nach Anspruch 1, wobei die Befehle mit Frequenzen von mehr als etwa 20 kHz übertragen werden, um Hintergrundgeräusche zu minimieren.

7. Das System nach Anspruch 2, wobei das Kommunikationselement (612) so ausgebildet ist, dass es ein oder mehrere voraufgezeichnete Befehle an den Hund sendet, wenn das aktive Geräuschreduzierungssystem (900) ausgeschaltet ist.

8. Das System nach Anspruch 1, wobei das System so ausgebildet ist, dass es das aktive Geräuschreduzierungssystem (900) ausschaltet, wenn von dem zumindest einen Mikrofon (602, 603) laute Impulsgeräusche erfasst werden.

9. Das System nach Anspruch 2, wobei das Kommunikationselement in einem Elektronikmodul (801) aufgenommen ist, welches so ausgebildet ist, dass es mit Hilfe eines Geschirrs (802) an den Hund geschnallt werden kann.

10. Das System nach Anspruch 9, wobei das Elektronikmodul (801) ferner eine Stromversorgung umfasst.

11. Das System nach Anspruch 10, wobei der Funkempfänger so ausgebildet ist, dass er in wählbaren Frequenzbereichen wie 30-512 MHz, 225-450 MHz, 1250-1390 MHz und 1750-1850 MHz arbeitet, um mit einem geeigneten Funkgerät zu kommunizieren.

12. Das System nach Anspruch 9, wobei der Funkempfänger so ausgebildet ist, dass er mit einem Bluetooth- und/oder WiFi-fähigen Kommunikationsgerät kommunizieren kann.

13. Das System nach Anspruch 1, ferner aufweisend zumindest einen Gehirnaktivitätsmonitor, einen Herzfrequenzsensor sowie einen Körpertemperatursensor, um die Reaktion des Hundes mit einem äußeren Ereignis zu korrelieren.

## Revendications

1. Un système de protection auditive et de communication (800) pour un chien, le système comportant :
un élément de réduction de bruit pour détecter et atténuer le bruit externe jusqu'à un niveau de seuil de bruit intra-auriculaire prédéterminé ;
une pluralité de comparateurs (604, 605) pour échantillonner le bruit externe et des données de bruit intra-auriculaire générées par l'élément de réduction de bruit et reliés à une commande (601) de manière opérationnelle, dans lequel la commande (601) est configurée pour commander le fonctionnement du système de protection auditive ; et
un élément de communication (612) relié à la commande (601) de manière opérationnelle et configuré pour permettre au maître-chien de communiquer avec le chien, dans lequel l'élément de communication (612) comporte un élément de contournement pour permettre au maître-chien de contourner des instructions de réduction de bruit et de transmission au chien à des fréquences supérieures à environ 10 kHz,
dans lequel le niveau de seuil de bruit intra-auriculaire prédéterminé est un niveau de bruit qui est inférieur au niveau de bruit externe de 20 dB à 30 dB, et
dans lequel l'élément de réduction de bruit comporte au moins un microphone (602, 603) pour capturer le bruit externe et le bruit intra-auriculaire, un élément de réduction passive du bruit, non linéaire, capable d'augmenter l'atténuation du bruit externe à mesure que le niveau de bruit externe augmente, et un sous-système d'annulation active du bruit (900).

2. Le système selon la revendication 1, dans lequel l'élément de communication (612) comporte au moins un élément parmi une radio, un récepteur radio, un dispositif de communication Bluetooth, un dispositif de communication sans fil, une antenne et une unité d' interface utilisateur.

3. Le système selon la revendication 1, dans lequel l'élément de communication (612) est en outre configuré pour déchiffrer des instructions chiffrées à l'oreille du chien jusqu'à la plage de fréquences audibles comprise entre environ 100 Hz et environ 4 kHz.

4. Le système selon la revendication 1, dans lequel le système comporte un système intra-auriculaire.

5. Le système selon la revendication 1, dans lequel le système comporte un système supra-auriculaire.

6. Le système selon la revendication 1, dans lequel des instructions sont transmises à des fréquences supérieures à environ 20 kHz pour minimiser le bruit de fond.

7. Le système selon la revendication 2, dans lequel l'élément de communication (612) est configuré pour transmettre au chien une ou plusieurs instructions pré-enregistrées lorsque le système d'annulation active du bruit (900) est arrêté.

8. Le système selon la revendication 1, dans lequel le système est configuré pour arrêter le système d'annulation active du bruit (900) lorsque des bruits impulsionnels forts sont détectés par le au moins un microphone (602, 603).

9. Le système selon la revendication 2, dans lequel l'élément de communication est reçu dans un module électronique (801) configuré pour être sanglé au chien en utilisant un harnais (802).

10. Le système selon la revendication 9, dans lequel le module électronique (801) comporte en outre une alimentation électrique.

11. Le système selon la revendication 10, dans lequel le récepteur radio est configuré pour fonctionner dans des plages de fréquences sélectionnables telles que 30-512 MHz, 225-450 MHz, 1 250-1 390 MHz et 1 750-1 850 MHz, pour communiquer avec une radio adaptée.

12. Le système selon la revendication 9, dans lequel le récepteur radio est configuré pour communiquer avec au moins un élément parmi un dispositif de communication compatible Bluetooth et un dispositif de communication compatible WiFi.

13. Le système selon la revendication 1, comportant en outre au moins un élément parmi un moniteur d'activité cérébrale, un capteur de fréquence cardiaque et un capteur de température corporelle pour corréler la réponse du chien à un événement externe.
